# EUROPÄISCHE PATENTSCHRIFT

(11) **EP 2 142 151 B1**
(45) Veröffentlichungstag und Bekanntmachung des Hinweises auf die Patenterteilung: **11.10.2017**
(21) Anmeldenummer: 08716444.8
(22) Anmeldetag: 11.03.2008
(51) Int. Cl.: A61F 9/008

(54) **VORRICHTUNG UND VERFAHREN ZUM ERZEUGEN VON SCHNITTFLÄCHEN IN DER HORNHAUT EINES AUGES ZUR FEHLSICHTIGKEITSKORREKTUR**
APPARATUS AND METHOD FOR THE GENERATION OF CUT AREAS IN THE CORNEA OF AN EYE FOR THE CORRECTION OF DEFECTIVE VISION
DISPOSITIF ET PROCÉDÉ POUR RÉALISER DES SURFACES DE COUPE DANS LA CORNÉE D'UN OEIL AFIN DE CORRIGER UNE AMÉTROPIE

(30) Priorität: 26.04.2007 DE 102007019813; 26.04.2007 US 914177 P
(43) Veröffentlichungstag der Anmeldung: 13.01.2010
(73) Patentinhaber: Carl Zeiss Meditec AG, 07745 Jena (DE)
(72) Erfinder: RUSSMANN, Christoph, 07745 Jena (DE); KUEHNERT, Jürgen, 07749 Jena (DE); BISSMANN, Wilfried, 07749 Jena (DE); STOBRAWA, Gregor, 07743 Jena (DE)
(74) Vertreter: Patentanwälte Geyer, Fehners & Partner mbB
(86) Internationale Anmeldenummer: PCT/EP2008/001937
(87) Internationale Veröffentlichungsnummer: WO 2008/131827

(56) Entgegenhaltungen:
- WO-A-2004/105661
- DE-A1-102005 049 281
- US-A- 5 970 984
- US-A- 5 993 438

## Beschreibung

Die Erfindung bezieht sich auf eine Vorrichtung zum Erzeugen von Schnittflächen in der Hornhaut eines Auges zur Fehlsichtigkeitskorrektur, mit einer Lasereinheit, die gepulste Laserstrahlung zur Schnittflächenerzeugung in die Hornhaut fokussieren und in dieser bewegen kann, und einer Steuereinheit, die die Lasereinheit zur Schnittflächenerzeugung so ansteuert, dass in der Hornhaut durch zumindest eine Schnittfläche ein vorbestimmtes, zu entnehmendes Lentikel vom umgebenden Hornhautmaterial getrennt wird und dass zumindest zwei voneinander beabstandete Schnittflächen als Öffnungsschnitte ausgeführt werden, die jeweils vom Lentikel bis zur Hornhautvorderseite verlaufen.

Ferner bezieht sich die Erfindung auf ein Verfahren zur Erzeugung von Steuerdaten für eine Steuereinheit einer Korrekturvorrichtung zum Erzeugen von Schnittflächen in der Hornhaut eines Auges zur Fehlsichtigkeitskorrektur, wobei die Korrekturvorrichtung
eine Lasereinheit, die gepulste Laserstrahlung zur Schnittflächenerzeugung in die Hornhaut fokussieren und in dieser bewegen kann, und eine Steuereinheit für die Lasereinheit aufweist, wobei solche Steuerdaten erzeugt werden, dass die Steuereinheit die Lasereinheit auf der Basis der Steuerdaten zur Schnittflächenerzeugung so ansteuern kann,
dass in der Hornhaut durch zumindest eine Schnittfläche ein vorbestimmtes, zu entnehmendes Lentikel vom umgebenden Hornhautmaterial getrennt wird und
dass zumindest zwei voneinander beabstandete Schnittflächen als Öffnungsschnitte ausgeführt werden, die jeweils vom Lentikel bis zur Hornhautvorderseite verlaufen.

Eine solche Vorrichtung und ein solches Verfahren sind beispielsweise aus der WO 2004/105661 A1 bekannt. Es hat sich inzwischen gezeigt, dass bei einer solchen Vorrichtung und einem solchen Verfahren häufig die gewünschte Fehlsichtigkeitskorrektur des Auges nicht erreicht wird.

Die US 5,970,984 beschreibt eine Verfahren, bei dem mehrere Schnitte in der Hornhaut zur Korrektur von Astigmatismus ausgeführt werden.

Ausgehend hiervon ist es Aufgabe der Erfindung, eine Vorrichtung der eingangs genannten Art so weiterzubilden, dass der beschriebene Nachteil möglichst vollständig überwunden werden kann. Ferner soll ein entsprechendes Verfahren der eingangs genannten Art entsprechend verbessert werden.

Die Erfindung ist in den Ansprüchen 1, 2, 3, 15, 16 und 17 definiert. Weiterbildungen sind in den abhängigen Ansprüchen angegeben.

Es hat sich nämlich gezeigt, dass die Öffnungsschnitte selbst, obwohl sie sehr klein sind, zu einer unerwünschten Verschlechterung der Fehlsichtigkeit des Auges führen. Da in der erfindungsgemäßen Vorrichtung dieser Effekt der Öffnungsschnitte nun erstmals berücksichtigt wird, kann vorteilhaft erreicht werden, dass die Öffnungsschnitte zur Korrektur der Fehlsichtigkeit beitragen oder der Korrektur der Fehlsichtigkeit des Auges nicht entgegenwirken, indem sie neutral bezüglich der zu korrigierenden Fehlsichtigkeit sind und/oder keine zusätzliche Fehlsichtigkeit verursachen. So kann z.B. bei der Korrektur von Kurzsichtigkeit oder Weitsichtigkeit verhindert werden, dass die Öffnungsschnitte einen unerwünschten Astigmatismus verursachen.

Bei der erfindungsgemäßen Vorrichtung zum Erzeugen von Schnittflächen in der Hornhaut eines Auges zur Fehlsichtigkeitskorrektur wird somit vorteilhaft ausgenutzt, dass ein vorzusehender Öffnungsschnitt, durch den das Lentikel entnommen werden kann, auch gleichzeitig hinsichtlich der Korrektur der Fehlsichtigkeit des Auges berücksichtigt wird. Es hat sich gezeigt, dass bei dem Vorsehen von zwei oder mehr Öffnungsschnitten sehr gute Korrekturergebnisse erzielt werden können. Ferner weist das Vorsehen von zwei oder mehr Öffnungsschnitten den Vorteil auf, dass diese beispielsweise zum Spülen des OP-Bereichs (Schnittbereich des Lentikels) genutzt werden können.

Ferner ist die Einbeziehung der Öffnungsschnitte für die Fehlsichtigkeitskorrektur dahingehend vorteilhaft, dass das zu entnehmende Lentikelvolumen minimiert werden kann. So sind Dickenreduzierungen von einigen µm bis zu 100 µm erreichbar. Damit kann beispielsweise eine mechanische Schwächung der Hornhaut, die durch die Material- bzw. Lentikelentnahme bedingt ist, so gering wie möglich gehalten werden.

Die Öffnungsschnitte sind so ausgeführt, dass ein Zurückklappen einer Hornhautlamelle wie bei der bekannten LASIK-Operation (Laser-Insitu-Keratomileusis) nicht möglich ist. Die Öffnungsschnitte führen somit zu keinem Flap, wie er bei der LASIK-Operation vorgesehen und zurückgeklappt wird.

Bei der zu korrigierenden Fehlsichtigkeit, kann es sich beispielsweise um Kurzsichtigkeit, Weitsichtigkeit, Astigmatismus und Altersichtigkeit handeln. Es ist auch möglich, dass Brechkraftfehler höherer Ordnungen korrigiert werden. Insbesondere Brechkraftfehler vierter Ordnung (sphärische Aberrationen) führen zu Problemen bei der Nachtsicht. Auch solche Brechkraftfehler der Hornhaut bzw. Fehlsichtigkeiten sind mit der erfindungsgemäßen Vorrichtung korrigierbar.

Die Steuereinheit kann die Lasereinheit insbesondere so ansteuern, dass zumindest drei voneinander beabstandete Öffnungsschnitte derart erzeugt werden, dass die Flächenschwerpunkte der Öffnungsschnitte mit den Ecken eines regelmäßigen Vielecks zusammenfallen. In diesem Fall ist es z.B. möglich, dass die Öffnungsschnitte keinen zusätzlichen Astigmatismus verursachen.

Die geometrische Form kann für jeden Öffnungsschnitt gleich sein. Es ist auch möglich, die geometrischen Formen unterschiedlich zu wählen. So kann beispielsweise die Steuereinheit die Lasereinheit so ansteuern, dass die Schnittlänge zumindest eines Öffnungsschnittes von der Hornhautvorderseite bis zum Lentikel verschieden von den Schnittlängen der anderen Öffnungsschnitte ausgeführt wird.

Ferner kann die Steuereinheit die Lasereinheit so ansteuern, dass genau zwei voneinander beabstandete Öffnungsschnitte derart erzeugt werden, dass die Flächenschwerpunkte der Öffnungsschnitte auf einer Geraden liegen, die in Draufsicht auf das Auge gesehen, parallel zur einen Astigmatismusachse des Auges verläuft oder diese unter einem Winkel von maximal 10° schneidet. Damit ist eine Astigmatismuskorrektur möglich. Bevorzugt wird die steilste Astigmatismusachse gewählt (also die Achse, zu der der größte Astigmatismus vorhanden ist).

Die Steuereinheit kann die Lasereinheit so ansteuern, dass zumindest einer der Öffnungsschnitte, in Draufsicht auf das Auge gesehen, die geometrische Form eines Kreisringabschnittes aufweist. Diese Form lässt sich besonders einfach mit herkömmlichen Lasereinheiten bei Augenkorrekturvorrichtungen verwirklichen.

Insbesondere kann die Steuereinheit die Lasereinheit so ansteuern, dass die gegenüberliegenden geraden Seiten des Kreisringabschnittes einen Winkel von 30° - 120°, bevorzugt 45° - 80° und besonders bevorzugt 30° - 60° einschließen.

Ferner kann die Steuereinheit die Lasereinheit so ansteuern, dass die gegenüberliegenden geraden Seiten des Kreisringabschnittes, in der Draufsicht gesehen eine Länge von 0,1 - 1 mm, bevorzugt 0,2 - 0,4 mm aufweisen.

Bei der erfindungsgemäßen Vorrichtung kann die Steuereinheit die Lasereinheit so ansteuern, dass eine weitere Schnittfläche als Entlastungsschnitt ausgeführt wird, der von der Hornhautvorderseite in die Hornhaut hinein aber nicht bis zum Lentikel verläuft, wobei Lage und Form des Entlastungsschnittes so gewählt ist, dass zur Korrektur der Fehlsichtigkeit des Auges beigetragen wird. Natürlich können mehrere Entlastungsschnitte vorgesehen werden. Durch den bzw. die zusätzlichen Entlastungsschnitte kann eine ausgezeichnete Fehlsichtigkeitskorrektur erreicht werden.

Die Steuereinheit kann die Lasereinheit so ansteuern, dass zumindest eine der Schnittflächen als perforierte Schnittfläche erzeugt wird. Unter einer perforierten Schnittfläche wird hier eine Schnittfläche verstanden, die nicht vollständig durchgehend ausgebildet ist, sondern die noch Materialbrücken aufweist, die bei einer vorbestimmten mechanischen Belastung (beispielsweise bei Entnahme des Lentikels) abreißen.

Mit der erfindungsgemäßen Vorrichtung kann ein Verfahren zum Erzeugen von Schnittflächen in der Hornhaut eines Auges zur Fehlsichtigkeitskorrektur durchgeführt werden, bei dem somit die Öffnungsschnitte zur Korrektur der Fehlsichtigkeit des Auges beitragen oder der Korrektur der Fehlsichtigkeit des Auges nicht entgegenwirken.

Mit dem Verfahren ist es möglich, die notwendig vorzusehenden Öffnungsschnitte gleich so auszuführen, dass sie zur Fehlsichtigkeitskorrektur beitragen oder der Korrektur der Fehlsichtigkeit nicht entgegenwirken. Damit ist es möglich, das Materialvolumen für das zu entfernende Lentikel zu minimieren. Insbesondere muss beispielsweise das Lentikel nicht so ausgebildet werden, dass es durch die Öffnungsschnitte bedingte Abbildungsfehler kompensiert, was nachteilig zu einem höheren, zu entfernenden Materialvolumen der Hornhaut führen würde.

Bei dem Verfahren kann die Lage und Form der Öffnungsschnitte so gewählt werden, dass der Korrektur der Fehlsichtigkeit des Auges derart nicht entgegengewirkt wird, dass die Öffnungsschnitte keine zusätzlichen Astigmatismus des Auges erzeugen. So ist es möglich, eine Kurzsichtigkeit oder Weitsichtigkeit zu korrigieren und dabei zu verhindern, dass der Patient nach Durchführung des Verfahrens zwar nicht mehr kurzsichtig bzw. weitsichtig ist, aber nun als Fehlsichtigkeit einen Astigmatismus des Auges hat.

Bei der zu korrigierenden Fehlsichtigkeit kann es sich um Kurzsichtigkeit, Weitsichtigkeit, Astigmatismus und/oder Altersweitsichtigkeit handeln. Die Fehlsichtigkeit kann ferner auch Brechkraftfehler höherer Ordnung der Hornhaut umfassen. Insbesondere kann es sich um Brechkraftfehler vierter Ordnung (sphärische Aberration) handeln, die für die Nachtsichtfähigkeit eine große Rolle spielt.

Bei dem Verfahren können zumindest drei voneinander beabstandete Öffnungsschnitte derart erzeugt werden, dass die Flächenschwerpunkte der Öffnungsschnitte mit den Ecken eines regelmäßigen Vieleckes zusammenfallen. In diesem Fall ist es z.B. möglich, dass die Öffnungsschnitte keinen zusätzlichen Astigmatismus verursachen.

Die Schnittlänge zumindest eines Öffnungsschnittes von der Hornhautvorderseite bis zum Lentikel kann verschieden von den Schnittlängen der anderen Öffnungsschnitte ausgeführt werden. Auch ist es möglich, dass die Öffnungsschnitte gleiche oder verschiedene geometrische Formen und/oder Abmessungen aufweisen.

Bei dem Verfahren können genau zwei voneinander beabstandete Öffnungsschnitte derart erzeugt werden, dass die Flächenschwerpunkte der Öffnungsschnitte auf einer Geraden liegen, die in Draufsicht auf das Auge gesehen, parallel zu einer Astigmatismusachse des Auges (bevorzugt die steilste Astigmatismusachse) verläuft oder diese unter einem Winkel von maximal 10° schneidet. Damit ist eine sehr gute Astigmatismuskorrektur möglich.

Die Öffnungsschnitte können so durchgeführt werden, dass zumindest einer der Öffnungsschnitte, in Draufsicht auf das Auge gesehen, die geometrische Form eines Kreisringabschnittes aufweist. Eine solche Form lässt sich mit einer Lasereinheit einer herkömmlichen Augenkorrekturvorrichtung in einfacher Art und Weise realisieren.

Insbesondere kann der Öffnungsschnitt so ausgeführt werden, dass die gegenüberliegenden geraden Seiten des Kreisringabschnittes einen Winkel von 30° - 120°, bevorzugt 45° - 80° und besonders bevorzugt 30° - 60° einschließen.

Ferner kann der zumindest eine Öffnungsabschnitt so ausgeführt werden, dass gegenüberliegende geraden Seiten des Kreisringabschnittes eine Länge von 0,1 - 1 mm, bevorzugt 0,2 - 0,4 mm aufweisen.

Bei dem Verfahren kann eine weitere Schnittfläche als Entlastungsschnitt ausgeführt werden, der von der Hornhautvorderseite in die Hornhaut hinein aber nicht bis zum Lentikel verläuft, wobei Lage und Form des Entlastungsschnittes so gewählt wird, dass er zur Korrektur der Fehlsichtigkeit des Auges beiträgt. Es können natürlich mehrere voneinander beabstandete Entlastungsschnitte durchgeführt werden. Mit dem bzw. den Öffnungsschnitten ist eine verbesserte Fehlsichtigkeitskorrektur möglich.

Bei dem Verfahren kann zumindest eine der Schnittflächen als perforierte Schnittfläche erzeugt werden. Dies kann im Ergebnis zu glatteren Schnittflächen im Vergleich zu Schnittflächen erzeugt werden, die als durchgehende Schnittflächen mittels der gepulsten Laserstrahlung erzeugt werden.

Insbesondere kann bei dem Verfahren das von dem umgebenden Hornhautmaterial getrennte Lentikel durch einen der Öffnungsschnitte aus der Hornhaut entfernt werden.

Ferner ist es möglich, das Lentikel mittels der gepulsten Laserstrahlung in zwei oder mehr Teile zu zerteilen und die Teile des Lentikels durch einen oder mehrere Öffnungsschnitte aus der Hornhaut zu entfernen.

Des weiteren können die Öffnungsschnitte auch zum Durchführen von einer Spülung der Schnittflächen oder gegebenenfalls zur Einbringung von Medikamenten verwendet werden.

Ferner wird ein Verfahren zur Erzeugung von Steuerdaten für eine Steuereinheit einer Korrekturvorrichtung zum Erzeugen von Schnittflächen in der Hornhaut eines Auges zur Fehlsichtigkeitskorrektur bereitgestellt, wobei die Korrekturvorrichtung eine Lasereinheit, die gepulste Laserstrahlung zur Schnittflächenerzeugung in die Hornhaut fokussieren und in diesen bewegen kann, und eine Steuereinheit für die Lasereinheit aufweist, wobei solche Steuerdaten erzeugt werden, dass die Steuereinheit die Lasereinheit auf der Basis der Steuerdaten zur Schnittflächenerzeugung so ansteuern kann, dass in der Hornhaut durch zumindest eine Schnittfläche ein vorbestimmtes, zu entnehmendes Lentikel vom umgebenden Hornhautmaterial getrennt wird und dass zumindest zwei voneinander beabstandete Schnittflächen als Öffnungsschnitte ausgeführt werden, die jeweils vom Lentikel bis zur Hornhautvorderseite verlaufen, wobei die Lage und Form der Öffnungsschnitte aufgrund der Steuerdaten so vorgegeben sind, dass die Öffnungsschnitte zur Korrektur der Fehlsichtigkeit des Auges beitragen oder der Korrektur der Fehlsichtigkeit des Auges nicht entgegenwirken. Das Verfahren zur Erzeugung von Steuerdaten kann so weitergebildet werden, dass die Weiterbildungen des Verfahrens zur Erzeugung von Schnittflächen in der Hornhaut eines Auges zur Fehlsichtigkeitskorrektur durchgeführt werden können.

Bei der Erzeugung der Schnittflächen gemäß der erfindungsgemäßen Vorrichtung und dem Verfahren in der Hornhaut durch die gepulste Laserstrahlung laufen im Gewebe zeitlich hintereinander mehrere Prozesse ab, die durch gepulste Laserstrahlung iniziiert werden. Ist die Leistungsdichte der Strahlung bei einem Impuls über einem Schwellwert, kommt es zu einem optischen Durchbruch, der in der Hornhaut beispielsweise eine Plasmablase erzeugt. Die Plasmablase wächst nach Entstehen des optischen Durchbruchs durch sich ausdehnendes Gas. Wird der optische Durchbruch nicht aufrechterhalten, so wird das in der Plasmablase erzeugte Gas vom umliegenden Gewebe aufgenommen und die Blase verschwindet wieder. Es sind auch Gewebetrenneffekte möglich, die ohne Plasmablase wirken. Der Einfachheit halber werden alle solche Prozesse einschließlich ihrer Wirkungen hier unter dem Begriff "optischer Durchbruch" zusammengefasst.

Zur Gewebetrennung wird die Laserstrahlung gepulst angewendet, wobei die Pulslänge in der Regel unter 1 ps liegt. Dadurch wird die zur Auslösung des optischen Durchbruchs nötige Leistungsdichte für den jeweiligen Puls in einem engen räumlichen Gebiet erreicht. Eine hohe Fokussierung des Laserstrahls in Kombination mit den kurzen Pulsen erlaubt es, den optischen Durchbruch punktgenau in der Hornhaut einzusetzen. Zur Schnitterzeugung wird eine Serie optischer Durchbrüche an den entsprechenden Stellen für die Schnittfläche erzeugt.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Ansicht einer Ausführungsform der erfindungsgemäßen Korrekturvorrichtung;
- Fig. 2: eine Draufsicht auf ein Auge nach Durchführung des Verfahrens zur Erzeugung von Schnittflächen in der Hornhaut eines Auges zur Fehlsichtigkeitskorrektur;
- Fig. 3: eine Querschnittsansicht der Hornhaut H entlang der Linie 17 von Fig. 2;
- Fig. 4: eine Draufsicht auf ein Auge nach Durchführung des Korrekturverfahrens gemäß einer weiteren Ausführungsform;
- Fig. 5: eine Draufsicht auf ein Auge nach Durchführung des Korrekturverfahrens gemäß einer weiteren Ausführungsform;
- Fig. 6: eine Draufsicht auf ein Auge nach Durchführung des Korrekturverfahrens gemäß einer weiteren Ausführungsform;
- Fig. 7: eine Draufsicht auf ein Auge nach Durchführung des Korrekturverfahrens gemäß einer weiteren Ausführungsform, und
- Fig. 8: eine Draufsicht auf ein Auge nach Durchführung des Korrekturverfahrens gemäß einer weiteren Ausführungsform.

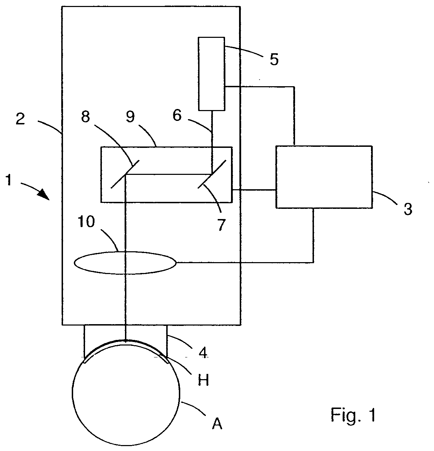

Bei der in Fig. 1 gezeigten Ausführungsform umfasst die Vorrichtung 1 zum Erzeugen von Schnittflächen in der Hornhaut H eines Auges A zur Fehlsichtigkeitskorrektur eine Lasereinheit 2 und eine Steuereinheit 3 zur Steuerung der Lasereinheit 2. Ferner kann die Vorrichtung 1, die nachfolgend auch Korrekturvorrichtung genannt wird, ein mit der Lasereinheit 2 lösbar gekoppeltes Kontaktelement 4 aufweisen, an dem das zu korrigierende Auge A beim Betrieb der Vorrichtung 1 anliegt.

Die Lasereinheit 2 umfasst, wie der schematischen Darstellung von Fig. 1 zu entnehmen ist, einen Laser 5, der gepulste Laserstrahlung 6 abgibt. Die Pulsdauer liegt dabei beispielsweise im Femtosekundenbereich (z.B. 50 - 800 fs) mit einer Pulswiederholungsfrequenz zwischen 10 und 1 MHz.

Die gepulste Laserstrahlung 6 wird mittels zweier Ablenkspiegel 7, 8, die einen Scanner 9 bilden, und einer Optik 10 durch das Kontaktelement 4 hindurch in die Hornhaut H eines am Kontaktelement 4 anliegenden Auges A fokussiert und in der Hornhaut H bewegt. Dies geschieht unter Steuerung der Steuereinheit 3, so dass grundsätzlich beliebige Orte in der Hornhaut mit der gepulsten Laserstrahlung 6 beaufschlagt werden können.

Der Scanner kann natürlich auch in anderer, dem Fachmann bekannter Weise ausgebildet sein.

Die Steuereinheit 3 kann die Lasereinheit 2 so ansteuern, dass an dem jeweiligen Fokusort in der Hornhaut H ein optischer Durchbruch zur Gewebetrennung erzeugt wird. Die Fokusorte werden so nebeneinanderliegend gewählt, dass eine gewünschte Schnittfläche in der Hornhaut H erzeugt werden kann. Dabei können die Fokusorte so nebeneinanderliegen, dass zwischen den Fokusorten das Gewebe vollständig durchtrennt wird. Es ist jedoch auch möglich, dass kleine Gewebebrücken verbleiben, so dass die Schnittfläche als perforierte Schnittfläche bezeichnet werden kann.

Die Lasereinheit 2 und die Steuereinheit 3 sind in Fig. 1 schematisch und stark vereinfacht dargestellt. So kann beispielsweise die Optik 10, die lediglich als Linse dargestellt ist, mehrere optische Elemente aufweisen, die entlang des Strahlengangs vom Laser 5 bis zum Kontaktelement 4 in geeigneter Weise angeordnet sind.

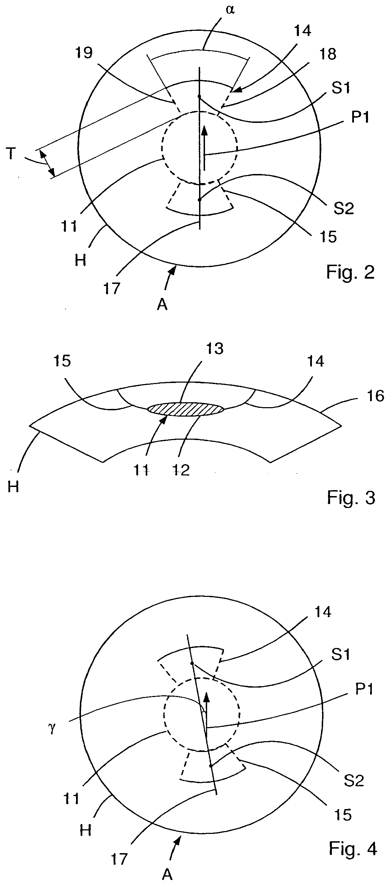

Die Korrekturvorrichtung 1 kann so betrieben werden, dass für die Fehlsichtigkeitskorrektur (hier z.B. Korrektur von Kurzsichtigkeit und Astigmatismus) ein bevorzugt linsenförmiges Teilvolumen 11 (nachfolgend auch als Lentikel bezeichnet) innerhalb der Hornhaut H von dem umgebenden Hornhautmaterial mittels der gepulsten Laserstrahlung 6 getrennt wird. Dies wird bevorzugt so durchgeführt, dass zuerst die Rückseite 12 (Fig. 3) des Lentikels 11 und danach die Vorderseite 13 des Lentikels 11 geschnitten wird. Um das von dem restlichen Hornhautmaterial getrennte Lentikel 11 aus der Hornhaut H entfernen zu können, werden, wie in Fig. 2 und 3 schematisch dargestellt ist, ein erster und ein zweiter Öffnungsschnitt 14, 15 durchgeführt, die jeweils vom Lentikel 11 bis zur Hornhautvorderseite 16 verlaufen.

Das Lentikel 11 kann dann in bekannter Weise durch den ersten oder zweiten Öffnungsschnitt 14, 15 hindurch aus der Hornhaut H entfernt werden. Aufgrund des fehlenden Hornhautvolumens (Lentikel 11) wird die Hornhaut in diesem Bereich ihre Form ändern. Die Form des Lentikels wurde vor der Durchführung des Verfahrens so gewählt, dass die Hornhaut nach Entfernen des Lentikels eine solche Form aufweist, dass die gewünschte Fehlsichtigkeitskorrektur erreicht ist.

In dem beschriebenen Ausführungsbeispiel von Fig. 2 und 3 wurden zwei Öffnungsschnitte 14 und 15 durchgeführt und die Form und Lage der Öffnungsschnitte 14 und 15 so gewählt, dass ein noch vorhandener Astigmatismus des Auges A gleich mit korrigiert wird.

Anders gesagt, es wird erfindungsgemäß berücksichtigt, dass selbst die sehr kleinen Öffnungsschnitte 14 und 15 zu einer Beeinflussung des Astigmatismus des Auges A führen. Dieser an sich unerwünschte Effekt wird erfindungsgemäß ausgenutzt, um einen vorhandenen Astigmatismus des Auges A zu korrigieren.

In dem beschriebenen Ausführungsbeispiel wird angenommen, dass die steilste Astigmatismusachse in Fig. 2 von oben nach unten verläuft, wie durch den Pfeil P1 angedeutet ist. In diesem Fall werden die beiden Öffnungsschnitte 14 und 15 so angeordnet, dass ihre Flächenschwerpunkte S1 und S2 auf einer Geraden 17 liegen, die parallel zur Astigmatismusachse P1 verläuft oder mit dieser zusammenfällt. In Fig. 2 ist lediglich zur Verdeutlichung der Darstellung die Astigmatismusachse P1 etwas seitlich neben der Geraden 17 dargestellt.

Durch diese Anordnung der Öffnungsschnitt 14 und 15 wird aufgrund der Öffnungsschnitte 14 und 15 der Astigmatismus des Auges A verringert.

Wie Fig. 2 zu entnehmen ist, sind die Öffnungsschnitte 14 und 15, in der Draufsicht von Fig. 2 gesehen, jeweils als Kreisringabschnitte ausgebildet. Da beide Öffnungsschnitte 14 und 15 gleich ausgebildet sind, wird nachfolgend nur der erste Öffnungsschnitt 14 detaillierter beschrieben.

Die beiden geraden Seiten 18 und 19 des Öffnungsschnittes 14 weisen eine gleiche Länge T (Fig. 2) auf, wobei diese Länge in dem hier beschriebenen Ausführungsbeispiel zwischen 0,1 und 1 mm liegt. Bevorzugt beträgt die Länge T 0,2 - 0,4 mm. Ferner schließen die beiden geraden Seiten 18 und 19 einen Winkel α ein, der im Bereich von 30° - 120° liegen kann. Bevorzugt beträgt er 40° - 80°.

In Fig. 4 ist eine Abwandlung der Ausführungsform von Fig. 2 und 3 gezeigt. Bei dieser Abwandlung werden die Öffnungsschnitte 14 und 15 so gelegt, dass die die Flächenschwerpunkte S1, S2 verbindende Gerade 17 mit der Astigmatismusachse P1 einen Winkel γ einschließt, der hier 10° beträgt. Auch bei dieser Abweichung wird noch eine ausgezeichnete Astigmatismuskorrektur durch die Öffnungssschnitte 14 und 15 erreicht.

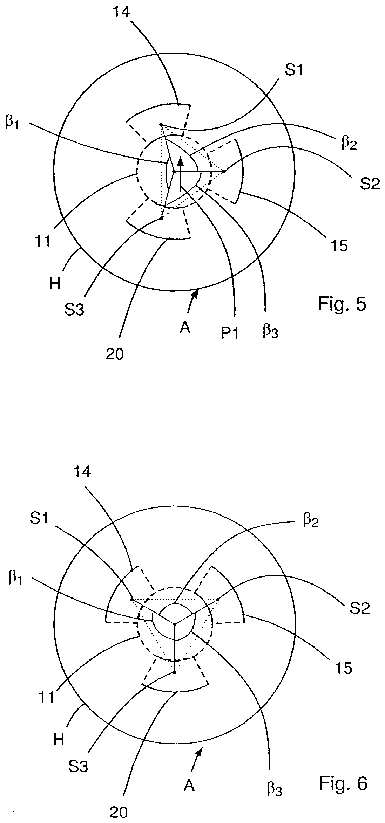

Wie bei der in Fig. 5 gezeigten Ausführungsform ersichtlich ist, wurden drei Öffnungsschnitte 14, 15 und 20 durchgeführt. Die Öffnungsschnitte 14, 15 und 20 sind voneinander beabstandet, wobei die Flächenschwerpunkte S1, S2 und S3 ein Dreieck aufspannen, das in Fig. 5 gepunktet eingezeichnet ist.

Um mittels der Öffnungsschnitte 14, 15 und 20 eine Astigmatismuskorrektur zu erzielen, sind die Öffnungsschnitte 14, 15 und 20, wie in Fig. 5 ersichtlich ist, am Umfang des Lentikels 11 mit ungleichem Winkelabstand voneinander angeordnet. So beträgt der Winkel β₁ 150° und die Winkel β₂ und β₃ jeweils 105°. Durch diese unsymmetrische winkelmäßige Verteilung der Öffnungsschnitte 14, 15 und 20 wird die gewünschte Astigmatismuskorrektur erreicht.

Es ist jedoch auch möglich, die Öffnungsschnitte 14, 15 und 20 winkelmäßig gleichmäßig zu verteilen, wie in Fig. 6 gezeigt ist. Hier betragen die Winkel β₁, β₂ und β₃ jeweils 120°. Diese Verteilung der Öffnungsschnitte wird gewählt, wenn keine Astigmatismuskorrektur mittels der Öffnungsschnitte 14, 15 und 20 gewünscht ist. Dies kann beispielsweise dann der Fall sein, wenn kein Astigmatismus zu korrigieren ist, sondern nur eine Kurzsichtigkeit. Somit können die Öffnungsschnitte 14, 15 und 20 so vorgesehen werden, dass keine Beeinflussung des Astigmatismus erfolgt. Wie in Fig. 6 ersichtlich ist, ist dann das durch die Flächenschwerpunkte S1, S2 und S3 aufgespannte Dreieck ein gleichseitiges Dreieck.

Ferner ist es möglich, dass die Öffnungsschnitte 14, 15 und 20 in Fig. 6 so angeordnet sind und eine solche Form aufweisen, dass sie zur gewünschten Korrektur der Kurzsichtigkeit beitragen.

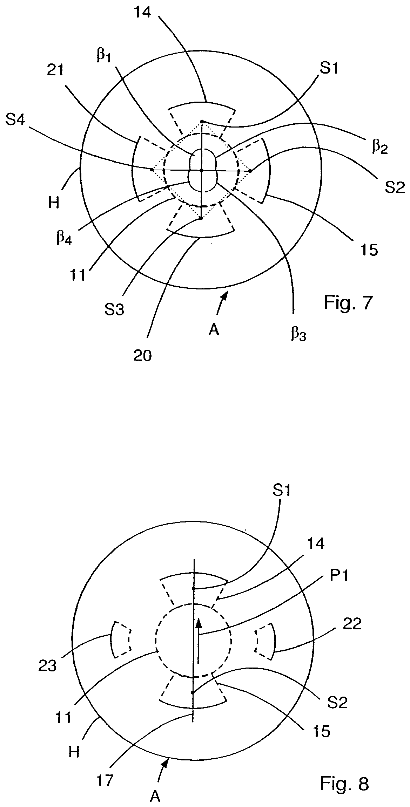

Ein Vorsehen der Öffnungsschnitte ohne Beeinflussung des Astigmatismus der Hornhaut H ist auch bei vier Öffnungsschnitten möglich, wie in Fig. 7 gezeigt ist. Die vier Öffnungsschnitte 14, 15, 20 und 21 sind wiederum winkelmäßig gleichmäßig um den Umfang des Lentikels 11 verteilt, so dass in diesem Fall die Flächenschwerpunkte S1, S2, S3 und S4 ein Quadrat aufspannen.

Allgemein kann man sagen, dass n Öffnungsschnitte (mit n > 2) so vorgesehen werden können, dass ihre Flächenschwerpunkte ein regelmäßiges n-Eck bilden, um keinerlei Astigmatismusbeeinflussung durch die n Öffnungsschnitte zu verursachen.

Wie in Fig. 8 angedeutet ist, in der eine Weiterbildung der Ausführungsformen von Fig. 2 und 3 dargestellt ist, können zusätzlich zu den Öffnungsschnitten 14, 15 Entlastungsschnitte 22, 23 durchgeführt werden, die sich von der Vorderseite der Hornhaut H in diese hinein erstrecken, jedoch nicht bis zum Lentikel 11 hin. Diese Entlastungsschnitte 22, 23 können zur Korrektur der mittels des Lentikels 11 zu korrigierenden Fehlsichtigkeit und/oder des Astigmatismus (Pfeil P1) eingesetzt werden.

## Patentansprüche

1. Vorrichtung zum Erzeugen von Schnittflächen in der Hornhaut eines Auges zur Fehlsichtigkeitskorrektur, mit
einer Lasereinheit (2), die gepulste Laserstrahlung (6) zur Schnittflächenerzeugung in die Hornhaut (H) fokussieren und in dieser bewegen kann, und
einer Steuereinheit (3), die die Lasereinheit (2) zur Schnittflächenerzeugung so ansteuert,
dass in der Hornhaut (H) durch zumindest eine Schnittfläche (12, 13) ein vorbestimmtes, zu entnehmendes Lentikel (11) vom umgebenden Hornhautmaterial getrennt wird und
dass zumindest zwei voneinander beabstandete Schnittflächen als Öffnungsschnitte (14, 15, 20, 21) ausgeführt werden, die jeweils vom Lentikel (11) bis zur Hornhautvorderseite (16) verlaufen,
**dadurch gekennzeichnet, dass** die Lage und Form der Öffnungsschnitte (14, 15, 20, 21) so gewählt ist, dass die Öffnungsschnitte (14, 15, 20, 21) zur Korrektur der Fehlsichtigkeit des Auges (A) beitragen oder der Korrektur der Fehlsichtigkeit des Auges (A) nicht entgegenwirken, wobei genau zwei voneinander beabstandete Öffnungsschnitte (14, 15) so erzeugt werden, dass die Flächenschwerpunkte (S1, S2) der Öffnungsschnitte auf einer Geraden (17) liegen, die in Draufsicht auf das Auge (A) gesehen, parallel zu einer Astigmatismusachse (P1) des Auges (A) verläuft oder diese unter einem Winkel (γ) von maximal 10° schneidet.

2. Vorrichtung zum Erzeugen von Schnittflächen in der Hornhaut eines Auges zur Fehlsichtigkeitskorrektur, mit
einer Lasereinheit (2), die gepulste Laserstrahlung (6) zur Schnittflächenerzeugung in die Hornhaut (H) fokussieren und in dieser bewegen kann, und
einer Steuereinheit (3), die die Lasereinheit (2) zur Schnittflächenerzeugung so ansteuert,
dass in der Hornhaut (H) durch zumindest eine Schnittfläche (12, 13) ein vorbestimmtes, zu entnehmendes Lentikel (11) vom umgebenden Hornhautmaterial getrennt wird und
dass zumindest zwei voneinander beabstandete Schnittflächen als Öffnungsschnitte (14, 15, 20, 21) ausgeführt werden, die jeweils vom Lentikel (11) bis zur Hornhautvorderseite (16) verlaufen,
**dadurch gekennzeichnet, dass** die Lage und Form der Öffnungsschnitte (14, 15, 20, 21) so gewählt ist, dass die Öffnungsschnitte (14, 15, 20, 21) zur Korrektur der Fehlsichtigkeit des Auges (A) beitragen oder der Korrektur der Fehlsichtigkeit des Auges (A) nicht entgegenwirken, wobei die Steuereinheit (3) die Lasereinheit (2) so ansteuert, dass eine weitere Schnittfläche (22, 23) als Entlastungsschnitt ausgeführt wird, der von der Hornhautvorderseite (16) in die Hornhaut (H) hinein aber nicht bis zum Lentikel (11) verläuft, wobei Lage und Form des Entlastungsschnittes (22, 23) so gewählt ist, dass zur Korrektur der Fehlsichtigkeit des Auges (A) beigetragen wird.

3. Vorrichtung zum Erzeugen von Schnittflächen in der Hornhaut eines Auges zur Fehlsichtigkeitskorrektur, mit
einer Lasereinheit (2), die gepulste Laserstrahlung (6) zur Schnittflächenerzeugung in die Hornhaut (H) fokussieren und in dieser bewegen kann, und
einer Steuereinheit (3), die die Lasereinheit (2) zur Schnittflächenerzeugung so ansteuert,
dass in der Hornhaut (H) durch zumindest eine Schnittfläche (12, 13) ein vorbestimmtes, zu entnehmendes Lentikel (11) vom umgebenden Hornhautmaterial getrennt wird und
dass zumindest zwei voneinander beabstandete Schnittflächen als Öffnungsschnitte (14, 15, 20, 21) ausgeführt werden, die jeweils vom Lentikel (11) bis zur Hornhautvorderseite (16) verlaufen,
**dadurch gekennzeichnet, dass** die Lage und Form der Öffnungsschnitte (14, 15, 20, 21) so gewählt ist, dass die Öffnungsschnitte (14, 15, 20, 21) zur Korrektur der Fehlsichtigkeit des Auges (A) beitragen oder der Korrektur der Fehlsichtigkeit des Auges (A) nicht entgegenwirken, wobei die Steuereinheit (3) die Lasereinheit (2) so ansteuert, dass zumindest drei voneinander beabstandete Öffnungsschnitte (14, 15, 20) so erzeugt werden, dass die Flächenschwerpunkte (S1, S2, S3) der Öffnungsschnitte (14, 15, 20) mit den Ecken eines regelmäßigen Vielecks zusammenfallen.

4. Vorrichtung nach einem der obigen Ansprüche, bei der die Lage und Form der Öffnungsschnitte (14, 15, 20, 21) so gewählt ist, dass der Korrektur der Fehlsichtigkeit des Auges derart entgegengewirkt wird, dass die Öffnungsschnitte (14, 15, 20, 21) keinen zusätzlichen Astigmatismus des Auges (A) erzeugen.

5. Vorrichtung nach einem der obigen Ansprüche, bei der die korrigierende Fehlsichtigkeit einen Astigmatismus des Auges (A) umfasst.

6. Vorrichtung nach einem der obigen Ansprüche, bei der die Lage und Form der Öffnungsschnitte (14, 15, 20, 21) so gewählt ist, dass sie Brechkraftfehler höherer Ordnung der Hornhaut (H) korrigieren.

7. Vorrichtung nach Anspruch 2, bei der die Steuereinheit (3) die Lasereinheit (2) so ansteuert, dass zumindest drei voneinander beabstandete Öffnungsschnitte (14, 15, 20) so erzeugt werden, dass die Flächenschwerpunkte (S1, S2, S3) der Öffnungsschnitte (14, 15, 20) mit den Ecken eines regelmäßigen Vielecks zusammenfallen.

8. Vorrichtung nach einem der obigen Ansprüche, bei der die Steuereinheit (3) die Lasereinheit (2) so ansteuert, dass die Schnittlänge zumindest eines Öffnungsschnittes (14, 15, 20, 21) von der Hornhautvorderseite (16) bis zum Lentikel (11) verschieden von den Schnittlängen der anderen Öffnungsschnitte (14, 15, 20, 21) ausgeführt wird.

9. Vorrichtung nach Anspruch 2, bei der die Steuereinheit (3) die Lasereinheit (2) so ansteuert, dass genau zwei voneinander beabstandete Öffnungsschnitte (14, 15) so erzeugt werden, dass die Flächenschwerpunkte (S1, S2) der Öffnungsschnitte auf einer Geraden (17) liegen, die in Draufsicht auf das Auge (A) gesehen, parallel zu einer Astigmatismusachse (P1) des Auges (A) verläuft oder diese unter einem Winkel (γ) von maximal 10° schneidet.

10. Vorrichtung nach einem der obigen Ansprüche, bei der die Steuereinheit (3) die Lasereinheit (2) so ansteuert, dass zumindest einer der Öffnungsschnitte (14, 15, 20, 21), in Draufsicht auf das Auge (A) gesehen, die geometrische Form eines Kreisringabschnittes aufweist.

11. Vorrichtung nach Anspruch 10, bei der die Steuereinheit (3) die Lasereinheit (2) so ansteuert, dass die gegenüberliegenden geraden Seiten (18, 19) des Kreisringabschnittes einen Winkel von 30° - 120°, bevorzugt 45° - 80° einschließen.

12. Vorrichtung nach Anspruch 10 oder 11, bei der die Steuereinheit (3) die Lasereinheit (2) so ansteuert, dass die gegenüberliegenden geraden Seiten (18, 19) des Kreisringsabschnittes eine Länge von 0,1 - 1 mm, bevorzugt 0,2 - 0,4 mm aufweisen.

13. Vorrichtung nach Anspruch 1 oder 3, bei der die Steuereinheit (3) die Lasereinheit (2) so ansteuert, dass eine weitere Schnittfläche (22, 23) als Entlastungsschnitt ausgeführt wird, der von der Hornhautvorderseite (16) in die Hornhaut (H) hinein aber nicht bis zum Lentikel (11) verläuft, wobei Lage und Form des Entlastungsschnittes (22, 23) so gewählt ist, dass zur Korrektur der Fehlsichtigkeit des Auges (A) beigetragen wird.

14. Vorrichtung nach einem der obigen Ansprüche, bei der die Steuereinheit (3) die Lasereinheit (2) so ansteuert, dass zumindest eine der Schnittflächen (12, 13, 14, 15, 20, 21, 22, 23) als perforierte Schnittfläche erzeugt wird.

15. Verfahren zur Erzeugung von Steuerdaten für eine Steuereinheit einer Korrekturvorrichtung zum Erzeugen von Schnittflächen in der Hornhaut eines Auges zur Fehlsichtigkeitskorrektur, wobei die Korrekturvorrichtung
eine Lasereinheit, die gepulste Laserstrahlung zur Schnittflächenerzeugung in die Hornhaut fokussieren und in dieser bewegen kann, und eine Steuereinheit für die Lasereinheit aufweist, wobei solche Steuerdaten erzeugt werden, dass die Steuereinheit die Lasereinheit auf der Basis der Steuerdaten zur Schnittflächenerzeugung so ansteuern kann,
dass in der Hornhaut durch zumindest eine Schnittfläche ein vorbestimmtes, zu entnehmendes Lentikel vom umgebenden Hornhautmaterial getrennt wird und
dass zumindest zwei voneinander beabstandete Schnittflächen als Öffnungsschnitte ausgeführt werden, die jeweils vom Lentikel bis zur Hornhautvorderseite verlaufen,
**dadurch gekennzeichnet, dass** die Lage und Form der Öffnungsschnitte aufgrund der Steuerdaten so vorgegeben sind, dass die Öffnungsschnitte zur Korrektur der Fehlsichtigkeit des Auges beitragen oder der Korrektur der Fehlsichtigkeit des Auges nicht entgegenwirken, wobei die Steuereinheit die Lasereinheit auf der Basis der Steuerdaten zur Schnittflächenerzeugung so ansteuern kann, dass genau zwei voneinander beabstandete Öffnungsschnitte (14, 15) so erzeugt werden, dass die Flächenschwerpunkte (S1, S2) der Öffnungsschnitte auf einer Geraden (17) liegen, die in Draufsicht auf das Auge (A) gesehen, parallel zu einer Astigmatismusachse (P1) des Auges (A) verläuft oder diese unter einem Winkel (γ) von maximal 10° schneidet.

16. Verfahren zur Erzeugung von Steuerdaten für eine Steuereinheit einer Korrekturvorrichtung zum Erzeugen von Schnittflächen in der Hornhaut eines Auges zur Fehlsichtigkeitskorrektur, wobei die Korrekturvorrichtung
eine Lasereinheit, die gepulste Laserstrahlung zur Schnittflächenerzeugung in die Hornhaut fokussieren und in dieser bewegen kann, und eine Steuereinheit für die Lasereinheit aufweist, wobei solche Steuerdaten erzeugt werden, dass die Steuereinheit die Lasereinheit auf der Basis der Steuerdaten zur Schnittflächenerzeugung so ansteuern kann,
dass in der Hornhaut durch zumindest eine Schnittfläche ein vorbestimmtes, zu entnehmendes Lentikel vom umgebenden Hornhautmaterial getrennt wird und
dass zumindest zwei voneinander beabstandete Schnittflächen als Öffnungsschnitte ausgeführt werden, die jeweils vom Lentikel bis zur Hornhautvorderseite verlaufen,
**dadurch gekennzeichnet, dass** die Lage und Form der Öffnungsschnitte aufgrund der Steuerdaten so vorgegeben sind, dass die Öffnungsschnitte zur Korrektur der Fehlsichtigkeit des Auges beitragen oder der Korrektur der Fehlsichtigkeit des Auges nicht entgegenwirken, wobei die Steuereinheit die Lasereinheit auf der Basis der Steuerdaten zur Schnittflächenerzeugung so ansteuern kann, dass eine weitere Schnittfläche (22, 23) als Entlastungsschnitt ausgeführt wird, der von der Hornhautvorderseite (16) in die Hornhaut (H) hinein aber nicht bis zum Lentikel (11) verläuft, wobei Lage und Form des Entlastungsschnittes (22, 23) so gewählt ist, dass zur Korrektur der Fehlsichtigkeit des Auges (A) beigetragen wird.

17. Verfahren zur Erzeugung von Steuerdaten für eine Steuereinheit einer Korrekturvorrichtung zum Erzeugen von Schnittflächen in der Hornhaut eines Auges zur Fehlsichtigkeitskorrektur, wobei die Korrekturvorrichtung
eine Lasereinheit, die gepulste Laserstrahlung zur Schnittflächenerzeugung in die Hornhaut fokussieren und in dieser bewegen kann, und eine Steuereinheit für die Lasereinheit aufweist, wobei solche Steuerdaten erzeugt werden, dass die Steuereinheit die Lasereinheit auf der Basis der Steuerdaten zur Schnittflächenerzeugung so ansteuern kann,
dass in der Hornhaut durch zumindest eine Schnittfläche ein vorbestimmtes, zu entnehmendes Lentikel vom umgebenden Hornhautmaterial getrennt wird und
dass zumindest zwei voneinander beabstandete Schnittflächen als Öffnungsschnitte ausgeführt werden, die jeweils vom Lentikel bis zur Hornhautvorderseite verlaufen,
**dadurch gekennzeichnet, dass** die Lage und Form der Öffnungsschnitte aufgrund der Steuerdaten so vorgegeben sind, dass die Öffnungsschnitte zur Korrektur der Fehlsichtigkeit des Auges beitragen oder der Korrektur der Fehlsichtigkeit des Auges nicht entgegenwirken, wobei die Steuereinheit die Lasereinheit auf der Basis der Steuerdaten zur Schnittflächenerzeugung so ansteuern kann, dass zumindest drei voneinander beabstandete Öffnungsschnitte (14, 15, 20) so erzeugt werden, dass die Flächenschwerpunkte (S1, S2, S3) der Öffnungsschnitte (14, 15, 20) mit den Ecken eines regelmäßigen Vielecks zusammenfallen.

## Claims

1. A device for generating cut surfaces in the cornea of an eye for correcting defective vision, comprising
a laser unit (2), which can focus pulsed laser radiation (6) into the cornea (H) and move it therein for generating cut surfaces, and
a control unit (3) which controls the laser unit (2) for generating cut surfaces such that a predetermined lenticle (11) which is to be removed is separated from the surrounding corneal material by at least one cut surface (12, 13) in the cornea (H), and
that at least two spaced-apart cut surfaces are conducted as opening cuts (14, 15, 20, 21), each extending from the lenticle (11) to the front side of the cornea (16),
**characterized in that** the position and shape of the opening cuts (14, 15, 20, 21) are selected such that the opening cuts (14, 15, 20, 21) contribute to the correction of the defective vision of the eye (A) or do not counteract the correction of the defective vision of the eye (A),
wherein exactly two spaced-apart opening cuts (14, 15) are generated such that the centers of area (S1, S2) of the opening cuts (14, 15) are located on a straight line (17), which extends parallel to an astigmatism axis (P1) of the eye (A) or crosses the axis at an angle (γ) of a maximum of 10°, as viewed in plan view of the eye (A).

2. A device for generating cut surfaces in the cornea of an eye for correcting defective vision, comprising
a laser unit (2), which can focus pulsed laser radiation (6) into the cornea (H) and move it therein for generating cut surfaces, and
a control unit (3), which controls the laser unit (2) for generating cut surfaces such that a predetermined lenticle (11) which is to be removed is separated from the surrounding corneal material by at least one cut surface (12, 13) in the cornea (H), and
that at least two spaced-apart cut surfaces are conducted as opening cuts (14, 15, 20, 21), each extending from the lenticle (11) to the front side of the cornea (16),
**characterized in that** the position and shape of the opening cuts (14, 15, 20, 21) are selected such that the opening cuts (14, 15, 20, 21) contribute to the correction of the defective vision of the eye (A) or do not counteract the correction of the defective vision of the eye (A),
wherein the control unit (3) controls the laser unit (2) such that a further cut surface (22, 23) is conducted as relief cut which extends from the front side of the cornea (16) into the cornea (H), but does not extend as far as the lenticle (11), wherein the position and shape of the relief cut (22, 23) are selected to contribute to the correction of the defective vision of the eye (A).

3. A device for generating cut surfaces in the cornea of an eye for correcting defective vision, comprising
a laser unit (2), which can focus pulsed laser radiation (6) into the cornea (H) and move it therein for generating cut surfaces, and
a control unit (3) which controls the laser unit (2) for generating cut surfaces such that a predetermined lenticle (11) which is to be removed is separated from the surrounding corneal material by at least one cut surface (12, 13) in the cornea (H), and
that at least two spaced-apart cut surfaces are conducted as opening cuts (14, 15, 20, 21), each extending from the lenticle (11) to the front side of the cornea (16),
**characterized in that** the position and shape of the opening cuts (14, 15, 20, 21) are selected such that the opening cuts (14, 15, 20, 21) contribute to the correction of the defective vision of the eye (A) or do not counteract the correction of the defective vision of the eye (A),
wherein the control unit (3) controls the laser unit (2) such that at least three spaced-apart opening cuts (14, 15, 20) are generated such that the centers of area (S1, S2, S3) of the opening cuts (14, 15, 20) coincide with the corners of a regular polygon.

4. The device according to any of the above claims, wherein the position and shape of the opening cuts (14, 15, 20, 21) is selected to counteract the correction of the defective vision of the eye such that the opening cuts (14, 15, 20, 21) do not generate an additional astigmatism of the eye (A).

5. The device according to any of the above claims, wherein the corrective defect vision includes an astigmatism of the eye (A).

6. The device according to any of the above claims, wherein the position and shape of the opening cuts (14, 15, 20, 21) are selected to correct higher order refractive power errors of the cornea (H).

7. The device according to claim 2, wherein the control unit (3) controls the laser unit (2) such that at least three spaced-apart opening cuts (14, 15, 20) are generated such that the surface focus points (S1, S2, S3) of the opening cuts (14, 15, 20) coincide with the corners of a regular polygon.

8. The device according to any of the above claims, wherein the control unit (3) controls the laser unit (2) such that the cut length of at least one opening cut (14, 15, 20, 21) from the corneal front side (16) to the lenticle (11) is carried out differently from the cut lengths of the other opening cuts (14, 15, 20, 21).

9. The device according to claim 2, wherein the control unit (3) controls the laser unit (2) such that exactly two spaced-apart opening cuts (14, 15) are generated such that the centers of area (S1, S2) of the opening cuts are located on a straight line (17) which extends parallel to an astigmatism axis (P1) of the eye (A) or crosses the axis at an angle (γ) of a maximum of 10°, as viewed in plan view of the eye (A).

10. The device according to any of the above claims, wherein the control unit (3) controls the laser unit (2) such that at least one of the opening cuts (14, 15, 20, 21) comprises the geometrical shape of a circular ring portion, as seen in plan view of the eye (A).

11. The device according to claim 10, wherein the control unit (3) controls the laser unit (2) such that the opposite straight sides (18, 19) of the circular ring portion include an angle of 30° - 120°, preferably 45° - 80°.

12. The device according to claim 10 or 11, wherein the control unit (3) controls the laser unit (2) such that the opposite straight sides (18, 19) of the circular ring portion have a length of 0.1 - 1 mm, preferably 0.2 - 0.4 mm.

13. The device according to claim 1 or 3, wherein the control unit (3) controls the laser unit (2) such that a further cut surface (22, 23) is carried out as a relief cut which extends from the corneal front side (16) into the cornea (H), but does not extend as far as the lenticle (11), the position and shape of the relief cut (22, 23) being chosen to contribute to the correction of the defective vision of the eye (A).

14. The device according to any of the above claims, wherein the control unit (3) controls the laser unit (2) such that at least one of the cut surfaces (12, 13, 14, 15, 20, 21, 22, 23) is generated as a perforated cut surface.

15. A method for generating control data for a control unit of a correction device for generating cut surfaces in the cornea of an eye for correction of defective vision, the correction device comprising
a laser unit which can focus pulsed laser radiation into the cornea and move it therein for generating cut surfaces, and comprising a control unit for the laser unit,
such control data being generated in such a way that the control unit can control the laser unit based on the control data for the generation of cut surfaces such that a predetermined lenticle which is to be removed is separated from the surrounding corneal material by at least one cut surface in the cornea, and
that at least two spaced-apart cut surfaces are conducted as opening cuts, each extending from the lenticle to the front side of the cornea,
**characterized in that** the position and shape of the opening cuts are predetermined due to the control data such that the opening cuts contribute to the correction of the defective vision of the eye or do not counteract the correction of the defective vision of the eye,
wherein the control unit is capable of controlling the laser unit based on the control data for the generation of cut surfaces such that exactly two spaced-apart opening cuts (14, 15) are generated such that the centers of area (S1, S2) of the opening cuts are located on a straight line (17) which extends parallel to an astigmatism axis (P1) of the eye (A) or crosses the axis at an angle (γ) of a maximum of 10°, as viewed in plan view of the eye (A).

16. A method for generating control data for a control unit of a correction device for generating cut surfaces in the cornea of an eye for correction of defective vision, the correction device comprising
a laser unit, which can focus pulsed laser radiation into the cornea and move it therein for generating cut surfaces, and comprising a control unit for the laser unit,
such control data being generated in such a way that the control unit can control the laser unit based on the control data for the generation of cut surfaces such that a predetermined lenticle which is to be removed is separated from the surrounding corneal material by at least one cut surface in the cornea, and
that at least two spaced-apart cut surfaces are conducted as opening cuts, each extending from the lenticle to the front side of the cornea,
**characterized in that** the position and shape of the opening cuts are predetermined due to the control data such that the opening cuts contribute to the correction of the defective vision of the eye or do not counteract the correction of the defective vision of the eye,
wherein the control unit is capable of controlling the laser unit based on the control data for the generation of cut surfaces such that a further cut surface (22, 23) is carried out as a relief cut which extends from the corneal front side (16) into the cornea (H), but does not extend as far as the lenticle (11), the position and shape of the relief cut (22, 23) being chosen to contribute to the correction of the defective vision of the eye (A).

17. A method for generating control data for a control unit of a correction device for generating cut surfaces in the cornea of an eye for correction of defective vision, the correction device comprising
a laser unit, which can focus pulsed laser radiation into the cornea and move it therein for generating cut surfaces, and comprising a control unit for the laser unit,
such control data being generated in such a way that the control unit can control the laser unit based on the control data for the generation of cut surfaces such that a predetermined lenticle which is to be removed is separated from the surrounding corneal material by at least one cut surface in the cornea, and
that at least two spaced-apart cut surfaces are conducted as opening cuts, each extending from the lenticle to the front side of the cornea,
**characterized in that** the position and shape of the opening cuts are predetermined due to the control data such that the opening cuts contribute to the correction of the defective vision of the eye or do not counteract the correction of the defective vision of the eye,
wherein the control unit is capable of controlling the laser unit based on the control data for the generation of cut surfaces such that at least three spaced-apart opening cuts (14, 15, 20) are generated such that the centers of area (S1, S2, S3) of the opening cuts (14, 15, 20) coincide with the corners of a regular polygon.

## Revendications

1. Dispositif destiné à produire des surfaces de coupe dans la cornée d'un oeil pour corriger une amétropie, avec
une unité laser (2) qui peut focaliser un rayonnement laser (6) pulsé destiné à la production de surfaces de coupe dans la cornée (H) et le déplacer dans celle-ci, et
une unité de commande (3) qui pilote l'unité laser (2) destinée à la production de surfaces de coupe de telle sorte qu'un lenticule (11) prédéfini à enlever est séparé de la substance cornéenne environnante par au moins une surface de coupe (12, 13) dans la cornée (H), et qu'au moins deux surfaces de coupe espacées l'une de l'autre sont réalisées en tant qu'entailles d'ouverture (14, 15, 20, 21) qui s'étendent respectivement du lenticule (11) jusqu'au côté avant (16) de la cornée,
**caractérisé en ce que** la position et la forme des entailles d'ouverture (14, 15, 20, 21) sont choisies de telle sorte que les entailles d'ouverture (14, 15, 20, 21) contribuent à la correction de l'amétropie de l'oeil (A) ou ne s'opposent pas à la correction de l'amétropie de l'oeil (A), précisément deux entailles d'ouverture (14, 15) espacées l'une l'autre étant produites de telle sorte que les centres de gravité de surfaces (S1, S2) des entailles d'ouverture sont situés sur une droite (17) qui, en vue de dessus sur l'oeil (A), est parallèle à un axe astigmatique (P1) de l'oeil (A) ou coupe cet axe en formant un angle (γ) de 10° maximum.

2. Dispositif destiné à produire des surfaces de coupe dans la cornée d'un oeil pour corriger une amétropie, avec
une unité laser (2) qui peut focaliser un rayonnement laser (6) pulsé destiné à la production de surfaces de coupe dans la cornée (H) et le déplacer dans celle-ci, et une unité de commande (3) qui pilote l'unité laser (2) destinée à la production de surfaces de coupe de telle sorte qu'un lenticule (11) prédéfini à enlever est séparé de la substance cornéenne environnante par au moins une surface de coupe (12, 13) dans la cornée (H), et
qu'au moins deux surfaces de coupe espacées l'une de l'autre sont réalisées en tant qu'entailles d'ouverture (14, 15, 20, 21) qui s'étendent respectivement du lenticule (11) jusqu'au côté avant (16) de la cornée, **caractérisé en ce que** la position et la forme des entailles d'ouverture (14, 15, 20, 21) sont choisies de telle sorte que les entailles d'ouverture (14, 15, 20, 21) contribuent à la correction de l'amétropie de l'oeil (A) ou ne s'opposent pas à la correction de l'amétropie de l'oeil (A),
l'unité de commande (3) pilotant l'unité laser (2) de telle sorte qu'une autre surface de coupe (22, 23) est réalisée en tant qu'entaille de décharge qui s'étend à partir du côté avant (16) de la cornée jusque dans l'intérieur de la cornée (H) mais pas jusqu'au lenticule (11), la position et la forme de l'entaille de décharge (22, 23) étant choisies de telle sorte qu'une contribution est apportée à la correction de l'amétropie de l'oeil (A).

3. Dispositif destiné à produire des surfaces de coupe dans la cornée d'un oeil pour corriger une amétropie, avec
une unité laser (2) qui peut focaliser un rayonnement laser (6) pulsé destiné à la production de surfaces de coupe dans la cornée (H) et le déplacer dans celle-ci, et
une unité de commande (3) qui pilote l'unité laser (2) destinée à la production de surfaces de coupe de telle sorte qu'un lenticule (11) prédéfini à enlever est séparé de la substance cornéenne environnante par au moins une surface de coupe (12, 13) dans la cornée (H), et qu'au moins deux surfaces de coupe espacées l'une de l'autre sont réalisées en tant qu'entailles d'ouverture (14, 15, 20, 21) qui s'étendent respectivement du lenticule (11) jusqu'au côté avant (16) de la cornée, **caractérisé en ce que** la position et la forme des entailles d'ouverture (14, 15, 20, 21) sont choisies de telle sorte que les entailles d'ouverture (14, 15, 20, 21) contribuent à la correction de l'amétropie de l'oeil (A) ou ne s'opposent pas à la correction de l'amétropie de l'oeil (A),
l'unité de commande (3) pilotant l'unité laser (2) de telle sorte qu'au moins trois entailles d'ouverture (14, 15, 20) espacées les unes des autres sont produites de telle sorte que les centres de gravité de surfaces (S1, S2, S3) des entailles d'ouverture (14, 15, 20) coïncident avec les coins d'un polygone régulier.

4. Dispositif selon l'une des revendications ci-dessus, dans lequel la position et la forme des entailles d'ouverture (14, 15, 20, 21) sont choisies de telle sorte qu'il existe une opposition à la correction de l'amétropie de l'oeil de telle sorte que les entailles d'ouverture (14, 15, 20, 21) ne provoquent aucun astigmatisme supplémentaire de l'oeil (A).

5. Dispositif selon l'une des revendications ci-dessus, dans lequel l'amétropie corrigée comprend un astigmatisme de l'oeil (A).

6. Dispositif selon l'une des revendications ci-dessus, dans lequel la position et la forme des entailles d'ouverture (14, 15, 20, 21) sont choisies de telle sorte qu'elles corrigent des erreurs de réfraction optique d'ordre supérieur de la cornée (H).

7. Dispositif selon la revendication 2, dans lequel l'unité de commande (3) pilote l'unité laser (2) de telle sorte qu'au moins trois entailles d'ouverture (14, 15, 20) espacées les unes des autres sont produites de telle sorte que les centres de gravité de surfaces (S1, S2, S3) des entailles d'ouverture (14, 15, 20) coïncident avec les coins d'un polygone régulier.

8. Dispositif selon l'une des revendications ci-dessus, dans lequel l'unité de commande (3) pilote l'unité laser (2) de telle sorte que la longueur d'entaille d'au moins une entaille d'ouverture (14, 15, 20, 21) à partir du côté avant (16) de la cornée jusqu'au lenticule (11) est réalisée différemment des longueurs d'entailles des autres entailles d'ouverture (14, 15, 20, 21).

9. Dispositif selon la revendication 2, dans lequel l'unité de commande (3) pilote l'unité laser (2) de telle sorte que précisément deux entailles d'ouverture (14, 15) espacées l'une l'autre sont produites de telle sorte que les centres de gravité de surfaces (S1, S2) des entailles d'ouverture sont situés sur une droite (17) qui, en vue de dessus sur l'oeil (A), est parallèle à un axe astigmatique (P1) de l'oeil (A) ou coupe cet axe en formant un angle (γ) de 10° maximum.

10. Dispositif selon l'une des revendications ci-dessus, dans lequel l'unité de commande (3) pilote l'unité laser (2) de telle sorte qu'au moins une des entailles d'ouverture (14, 15, 20, 21), en vue de dessus sur l'oeil (A), présente la forme géométrique d'une entaille annulaire circulaire.

11. Dispositif selon la revendication 10, dans lequel l'unité de commande (3) pilote l'unité laser (2) de telle sorte que les côtés droits opposés (18, 19) du segment annulaire circulaire forment un angle de 30° - 120°, de préférence de 45° - 80°.

12. Dispositif selon la revendication 10 ou 11, dans lequel l'unité de commande (3) pilote l'unité laser (2) de telle sorte que les côtés droits opposés (18, 19) du segment annulaire circulaire présentent une longueur de 0,1 - 1 mm, de préférence de 0,2 - 0,4 mm.

13. Dispositif selon la revendication 1 ou 3, dans lequel l'unité de commande (3) pilote l'unité laser (2) de telle sorte qu'une autre surface de coupe (22, 23) est réalisée en tant qu'entaille de décharge qui s'étend à partir du côté avant (16) de la cornée jusque dans l'intérieur de la cornée (H) mais pas jusqu'au lenticule (11), la position et la forme de l'entaille de décharge (22, 23) étant choisies de telle sorte qu'une contribution est apportée à la correction de l'amétropie de l'oeil (A).

14. Dispositif selon l'une des revendications ci-dessus, dans lequel l'unité de commande (3) pilote l'unité laser (2) de telle sorte qu'au moins une des surfaces de coupe (12, 13, 14, 15, 20, 21, 22, 23) est produite en tant que surface de coupe perforée.

15. Procédé destiné à la production de données de commande pour une unité de commande d'un dispositif de correction destiné à la production de surfaces de coupe dans la cornée d'un oeil en vue de la correction de l'amétropie,
le dispositif de correction
présentant une unité laser qui peut focaliser un rayonnement laser pulsé destiné à la production de surfaces de coupe dans la cornée et le déplacer dans celle-ci, et une unité de commande pour l'unité laser, les données de commande produites étant telles que l'unité de commande peut piloter l'unité laser sur la base des données de commande destinées à la production de surfaces de coupe
de telle sorte qu'un lenticule prédéfini à enlever est séparé de la substance cornéenne environnante par au moins une surface de coupe dans la cornée, et
en ce qu'au moins deux surfaces de coupe espacées l'une de l'autre sont réalisées en tant qu'entailles d'ouverture qui s'étendent respectivement du lenticule jusqu'au côté avant de la cornée,
**caractérisé en ce que** la position et la forme des entailles d'ouverture sont spécifiées sur la base des données de commande de telle sorte que les entailles d'ouverture contribuent à la correction de l'amétropie de l'oeil ou ne s'opposent pas à la correction de l'amétropie de l'oeil,
l'unité de commande pouvant piloter l'unité laser sur la base des données de commande destinées à la production de surfaces de coupe de telle sorte que précisément deux entailles d'ouverture (14, 15) espacées l'une de l'autre sont produites de telle sorte que les centres de gravité de surfaces (S1, S2) des entailles d'ouverture sont situés sur une droite (17) qui, en vue de dessus sur l'oeil (A), est parallèle à un axe astigmatique (P1) de l'oeil (A) ou coupe cet axe en formant un angle (γ) de 10° maximum.

16. Procédé destiné à la production de données de commande pour une unité de commande d'un dispositif de correction destiné à la production de surfaces de coupe dans la cornée d'un oeil en vue de la correction de l'amétropie, le dispositif de correction présentant une unité laser qui peut focaliser un rayonnement laser pulsé destiné à la production de surfaces de coupe dans la cornée et le déplacer dans celle-ci, et une unité de commande pour l'unité laser, les données de commande produites étant telles que l'unité de commande peut piloter l'unité laser sur la base des données de commande destinées à la production de surfaces de coupe
de telle sorte qu'un lenticule prédéfini à enlever est séparé de la substance cornéenne environnante par au moins une surface de coupe dans la cornée, et
en ce qu'au moins deux surfaces de coupe espacées l'une de l'autre sont réalisées en tant qu'entailles d'ouverture qui s'étendent respectivement du lenticule jusqu'au côté avant de la cornée,
**caractérisé en ce que** la position et la forme des entailles d'ouverture sont spécifiées sur la base des données de commande de telle sorte que les entailles d'ouverture contribuent à la correction de l'amétropie de l'oeil ou ne s'opposent pas à la correction de l'amétropie de l'oeil,
l'unité de commande pouvant piloter l'unité laser sur la base des données de commande destinées à la production de surfaces de coupe de telle sorte qu'une autre surface de coupe (22, 23) est réalisée en tant qu'entaille de décharge qui s'étend à partir du côté avant (16) de la cornée jusque dans l'intérieur de la cornée (H) mais pas jusqu'au lenticule (11), la position et la forme de l'entaille de décharge (22, 23) étant choisies de telle sorte qu'une contribution est apportée à la correction de l'amétropie de l'oeil (A).

17. Procédé destiné à la production de données de commande pour une unité de commande d'un dispositif de correction destiné à la production de surfaces de coupe dans la cornée d'un oeil en vue de la correction de l'amétropie, le dispositif de correction présentant une unité laser qui peut focaliser un rayonnement laser pulsé destiné à la production de surfaces de coupe dans la cornée et le déplacer dans celle-ci, et une unité de commande pour l'unité laser, les données de commande produites étant telles que l'unité de commande peut piloter l'unité laser sur la base des données de commande destinées à la production de surfaces de coupe
de telle sorte qu'un lenticule prédéfini à enlever est séparé de la substance cornéenne environnante par au moins une surface de coupe dans la cornée, et
en ce qu'au moins deux surfaces de coupe espacées l'une de l'autre sont réalisées en tant qu'entailles d'ouverture qui s'étendent respectivement du lenticule jusqu'au côté avant de la cornée,
**caractérisé en ce que** la position et la forme des entailles d'ouverture sont spécifiées sur la base des données de commande de telle sorte que les entailles d'ouverture contribuent à la correction de l'amétropie de l'oeil ou ne s'opposent pas à la correction de l'amétropie de l'oeil,
l'unité de commande pouvant piloter l'unité laser sur la base des données de commande destinées à la production de surfaces de coupe de telle sorte qu'au moins trois entailles d'ouverture (14, 15, 20) espacées les unes des autres sont produites de telle sorte que les centres de gravité de surfaces (S1, S2, S3) des entailles d'ouverture (14, 15, 20) coïncident avec les coins d'un polygone régulier.
